# EUROPÄISCHE PATENTSCHRIFT

(11) **EP 0 946 563 B1**
(45) Veröffentlichungstag und Bekanntmachung des Hinweises auf die Patenterteilung: **17.07.2002**
(21) Anmeldenummer: 97951087.2
(22) Anmeldetag: 21.11.1997
(51) Int. Cl.: C07D 487/22, A61K 31/40

(54) **VERFAHREN ZUR GEWINNUNG VON HÄMIN AUS SCHLACHTBLUT**
METHOD OF OBTAINING HAEMIN FROM SLAUGHTER BLOOD
PROCEDE DE RECUPERATION D'HEMINE A PARTIR DE SANG D'ABATTAGE

(30) Priorität: 20.12.1996 DE 19653482; 30.09.1997 DE 19743330
(43) Veröffentlichungstag der Anmeldung: 06.10.1999
(73) Patentinhaber: FRAUNHOFER-GESELLSCHAFT ZUR FÖRDERUNG DER ANGEWANDTEN FORSCHUNG E.V., 80636 München (DE)
(72) Erfinder: LUCK, Thomas, D-80992 München (DE); MÄURER, Andreas, D-85354 Freising (DE)
(86) Internationale Anmeldenummer: DE9702748
(87) Internationale Veröffentlichungsnummer: WO9828302

(56) Entgegenhaltungen:
- EP-A- 0 013 055
- EP-A- 0 068 537
- EP-A- 0 236 971
- FR-A- 2 274 230

## Beschreibung

Aus der Literatur (z.B. Allgemeine und spezielle Pharmakologie und Toxikologie, Hrsg. W. Forth, D. Henschler, et al., 5. Aufl., Bl Wissenschaftsverlag, Mannheim, 1987, S. 389 ff.) ist bekannt, daß Hämeisen zur Eisenfortifikation geeignet ist. Der Vorteil von Hämeisen im Gegensatz zu dem in der Nahrung enthaltenen Eisen ist darin zu sehen, daß das Eisen in der Nahrung überwiegend als Non-Hämeisen vorliegt, dessen Verfügbarkeit durch pflanzliche Nahrungsliganden (Phytate, Oxalate, Tanine usw.) herabgesetzt ist. Diesen Resorptionseinschränkungen unterliegt Hämeisen nicht, das zudem in hohen Dosen die Darmschleimhaut erheblich weniger reizt und dadurch besser verträglich ist.

In der Literatur ist allerdings bisher noch kein Verfahren bekannt geworden, mit dem Häminpräparate mit einem entsprechend hohen Gehalt an Globin hergestellt werden können. Aus Ital. J. Gastroenterol. 27, 167, 1995, "Intestinal transfer of different haemin preparations in vitro", einem Artikel von K. Schümann und A. Mäurer, et al. ist lediglich bekannt geworden, daß Hämeisen zur Eisenfortifikation geeignet ist, und daß es durch Säurehydrolyse hergestellt werden kann.

Ausgehend hiervon, ist es die Aufgabe der vorliegenden Erfindung, ein Verfahren zur Gewinnung von Hämeisen vorzuschlagen, das zur Eisenfortifikation geeignet ist, wobei das hergestellte Häminpräparat einen hohen Gehalt an Globin (Protein) enthalten soll.

Die Aufgabe wird durch die kennzeichnenden Merkmale des Patentanspruchs 1 gelöst. Die Unteransprüche zeigen vorteilhafte Weiterbildungen auf.

Erfindungsgemäß wird somit vorgeschlagen, ausgehend von Schlachtblut, eine Säurebehandlung unter genau definierten Bedingungen durchzuführen. Erfindungsgemäß wird vorgeschlagen, das Schlachtblut zur Trennung des Hämins vom Globinanteil einer Säurebehandlung bei pH 1,2 bis 3 bei 45 bis 80 °C zu unterziehen. Es hat sich herausgestellt, daß nur unter diesen genau bestimmten Bedingungen ein entsprechendes Häminpräparat erhalten wird.

Beim erfindungsgemäßen Verfahren ist es dabei bevorzugt, daß vor der Säurebehandlung eine Trennung des Schlachtbluts in Dickblut und eine Plasmafraktion vorgenommen wird, und daß dann die Säurebehandlung nur beim Dickblut durchgeführt wird.

Besonders bevorzugt ist es, wenn die Säurebehandlung bei einem pH von 1,3 bis 2,0 und bei einer Temperatur von 60 bis 75 °C erfolgt. Beim erfindungsgemäßen Verfahren ist es weiterhin bevorzugt, wenn das Hämin vom Globin erst nach einer bestimmten Agglomerationszeit, bevorzugt nach einer Zeit von 15 Minuten bis 2 Stunden, besonders bevorzugt nach 30 Minuten bis 1,5 Stunden, abgetrennt wird. Es hat sich weiterhin gezeigt, daß es zur Maximierung des Hämingehalts im Präparat vorteilhaft ist, mit relativ verdünnten Lösungen (bevorzugter Konzentrationsbereich 2 bis 10 %) und verlängerten Aggregationszeiten (bis 1 Stunde) zu arbeiten. Dadurch erhöht sich auch gleichzeitig die Häminabtrennbarkeit und Ausbeute pro Fällansatz, so daß bei verbesserter Produktqualität kein Durchsatzverlust eintritt. Als optimalste Parameter für die Säurespaltung und anschließende Polymerisation des Hämins wurden ermittelt: Hämin-Globin-Konzentration 3,5 %, pH-Wert 1,5, Temperatur 72 °C und Reaktionszeit 50 Minuten.

Eine weitere Verfahrensvariante sieht vor, daß vor der Hämintrocknung die jeweils nach dem letzten Verfahrensschritt durchgeführt werden muß, eine Dissoziation der Häminaggregate durch Ultraschall erfolgt, um die gute Bioverfügbarkeit weiter zu verbessern. Dazu kann das feuchte Häminprodukt für 5 bis 60 Minuten, bevorzugt für 15 Minuten, im Ultraschallbad behandelt und anschließend im Gefriertrockner getrocknet werden.

Der Vorteil des erfindungsgemäßen Verfahrens besteht insbesondere darin, daß eine Verwertung des Kosten verursachenden Abfallproduktes Schlachtblut zu einer entfärbten Globinfraktion und einem eisenhaltigen Häminprodukt möglich wird. Das mit dem erfindungsgemäßen Verfahren hergestellte Häminprodukt weist zudem eine hohe Eisenbioverfügbarkeit auf und eröffnet folgende Anwendungsmöglichkeiten:
- Zusatzstoff zur Eisenfortifikation von pflanzlicher Nahrung für Menschen (Lebensmittelzusatzstoff, insbesondere für Dritte-Welt-Diät) und von Tierfutter (z.B. bei der Schweinemast),
- Pharmazeutische Eisenpräparate mit guter Verträglichkeit.

Das erfindungsgemäße Verfahren hat weiterhin den Vorteil, daß durch die gewählten Prozeßbedingungen eine wirksame Inaktivierung möglicherweise vorhandener pathogener Bakterien/Keime und Viren im Schlachtblut eintritt. Außerdem werden die Produktqualität mindernde Abbauenzyme (Proteasen etc.) inaktiviert. Aufgrund der erhalten gebliebenen natürlichen Bindung des Eisens im Hämin (Komplexierung durch Protoporphyrin-Ring) erweist sich das starke katalytische Potential des Eisens bezüglich Fettsäureoxidation und Aminsäureabbau als unbedenklich.

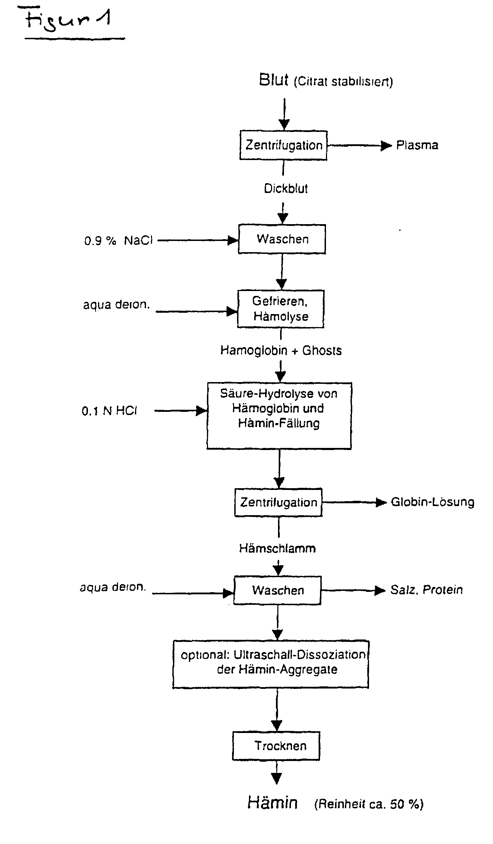

Die Erfindung wird nachfolgend anhand der Figur 1 und mehreren Ausführungsbeispielen näher erläutert.
- Fig. 1: zeigt beispielhaft den Verfahrensablauf zur Gewinnung von Hämin mit einer Reinheit von > 50 % für den Fall, daß vor der Säurebehandlung eine Auftrennung in Dickblut und eine Plasmafraktion erfolgt.

Wie aus Figur 1 hervorgeht, wird demnach das Blut, das vorteilhafterweise mit Citrat stabilisiert ist, zentrifugiert und dann die Plasmafraktion von dem Dickblut getrennt. Nach einem Waschschritt mit Kochsalzlösung wird das Dickblut eingefroren.

Zur Säurehydrolyse wird das so hergestellte Dickblut mit einer 0,1 normalen HCl versetzt, so daß eine Häminfällung eintritt. Zur Abtrennung der Globinlösug vom Hämschlamm wird eine Zentrifugation durchgeführt. Der Hämschlamm wird dann mit demineralisiertem Wasser gewaschen und ggf. einer Ultraschallbehandlung unterzogen. Zum Abschluß erfolgt eine Trocknung.

### Herstellungsbeispiele:

### 1. Kleiner Laboransatz

50 ml Blut werden 15 Minuten bei 3.300 g zentrifugiert. Es bilden sich 12 ml Dickblut (Erythrozyten) und 38 ml farbloses Blutplasma. Die Erythrozyten werden zweimal mit je 40 ml 0,9 %iger NaCl nachgewaschen und zentrifugiert. Das gewaschene Dickblut (20 g) wird mit 30 g deionisiertem Wasser aufgefüllt und bei -70 °C schockgefroren. Nach 3 Stunden wird es bei Raumtemperatur aufgetaut, die Erythrozytenmembranen (Ghosts) abzentrifugiert und der Überstand, die Hämoglobinlösung, in einen 250 ml Erlenmeyerkolben überführt. Auf einem Magnetrührer mit Heizplatte wird die tiefrote Lösung mit 1 normaler HCl auf pH 1,5 eingestellt und nach Erreichen von 71 °C für 30 min unter schwachem Rühren (150 Umin⁻¹) bei der Temperatur gehalten. Nach Abkühlen ohne Rühren bildet sich ein Sediment, der Hämschlamm, der in der Zentrifuge für 15 min bei 3.300 g abzentrifugiert wird. Dreimaliges Nachwaschen mit 0,1 normaler HCl und erneutes Zentrifugieren ergibt das Häminprodukt mit etwa 40 % Hämin-Gehalt und 70 % Ausbeute.

Optional erfolgt vor der Hämintrocknung eine Dissoziation der Häminaggregate durch Ultraschall, um die gute Bioverfügbarkeit weiter zu verbessern. Dazu wird das feuchte Häminprodukt für 15 min im Ultraschallbad behandelt und anschließend im Gefriertrockner getrocknet.

### 2. Großer Laboransatz

In einem 15 l Rührreaktor mit dampfbeheiztem Doppelmantel werden 8 l deionisiertes Wasser auf 67 °C erhitzt, mit ca. 1,5 l 1 normaler HCl auf pH 1,4 eingestellt und mit 2 kg tiefgefrorenem (stückigem) Dickblut versetzt. Die Temperatur wird auf 67 °C gehalten, der pH-Wert auf 2 eingestellt und mit 250 Umdrehungen pro Minute 1 Stunde lang gerührt. Nach Abschalten des Rührers erfolgt die Separation des Hämschlamms in einem Tellerseparator (Westfalia TA1) bei 9.000 - 10.000 U/min mit einem Durchsatz von 0,2 1/min. Das feuchte Produkt (Trockensubstanzgehalt 10 %) wird ohne Nachwaschen im Gefriertrockner oder alternativ im Umluft-Bandtrockner bei 80 °C Zulufttemperatur getrocknet (Reinheit: 30 % Hämingehalt; Ausbeute: 55 % der Theorie).

In der abgetrennten Globinlösung sind 0,06 % Hämin und 3,4 % teilweise lysiertes Protein enthalten. Bezogen auf die abgetrennte Menge bedeutet das einerseits einen Häminverlust von 45 % und zeigt andererseits, daß durch anschließendes Nachwaschen eine gute nachträgliche Häminreinigung (Globinabtrennung) möglich ist.

### Derivatherstellung:

Analoge Ansätze werden in einem 5 1-Becherglas auf einem Magnetrührer mit Heizplatte mit jeweils 500 g gefrorenem Hämschlamm und mit 2 l Ascorbinsäure, bzw. 2 l Citronensäure, bzw. 2 l Essigsäure durchgeführt. Die Reaktionstemperatur beträgt 70 °C, der eingestellte pH-Wert ist 2,3 (für Ascorbat- und Citratansatz) bzw. 1,7 (Essigsäure). Bezüglich Ausbeute, Hämingehalt und Bioverfügbarkeit ergeben sich keine signifikanten Unterschiede.

### 3. Ansatz im Technikumsmaßstab:

10 kg tiefgefrorenes, stückiges Dickblut (Hämoglobingehalt ca. 35 %) werden in einem 150 1 Rührbehälter mit direkter Dampfheizung unter schwachem Rühren (150 Umin⁻¹) in 90 kg vorgeheizte (75 °C) 0,01 n Salzsäure zugefügt. Der pH-Wert wird durch automatische Säuredosierung mit 6 n Salzsäure bei pH 1,6 konstant gehalten. Es erfolgt die Hämolyse, Säurespaltung der Hämin-Globin-Bindung, Häminaggregation und Sedimentation des Hämschlamms. Nach 1 Stunde Reaktionszeit bei 70 %C wird die Suspension mittels eines Dekanters aufgetrennt und der feuchte Hämmschlamm (Trockensubstanzgehalt 12 %) mit einem Bandtrockner bei 80 °C Zulufttemperatur getrocknet. Das hergestellte Produkt enthält etwa 40 % Hämin (Ausbeute 65 % der Theorie).

Die Hämin-Präparationen im 1000 Liter-Maßstab erfolgen in einem indirekt dampfbeheizten 3000 l Rührbehälter. Die Versuchsparametr entsprechen dem obigen Ansatz. Die eingesetzten Mengen sind 10-fach größer.

100 kg tiefgefrorenes, stückiges Dickblut (Hämoglobingehalt ca. 35 %) werden in einem 3000 l Rührbehälter mit indirekter Dampfheizung unter schwachem Rühren (150 Umin⁻¹) in 900 kg vorgeheizte (75 °C) 0,01 n Salzsäure zugefügt. Der pH-Wert wird durch automatische Säuredosierung mit 6 n Salzsäure bei pH 1,6 konstant gehalten. Es erfolgt die Hämolyse, Säurespaltung der Hämin-Globin-Bindung, Häminaggregation und Sepimentation des Hämschlamms. Nach einer Stunde Reaktionszeit bei 70 °C wird die Suspension mittels eines Dekanters aufgetrennt und der feuchte Hämschlamm (Trockensubstanzgehalt 12 %) mit einem Bandtrockner bei 80 °C Zulufttemperatur getrocknet. Das hergestellte Produkt enthält etwa 40 % Hämin (ausbeute 65 % der Theorie).

Für eine großtechnische Häminpräparation ist es sinnvoll, auch den diskontinuierlichen Fällschritt in einen kontinuierlichen Prozeß zu wandeln. Dabei lassen sich durch einen Kreislaufbetrieb der heißen, sauren Globinlösung zusätzlich Energie, Rohstoffe und Abwasserkosten einsparen. Dazu erfolgt die Zugabe und das Mischen der verdünnten Säure mit dem Dickblut in einem separaten Apparat (Rührbehälter) und die anschließende Häminpolymerisation und Fällung in einem Rohr- oder Rohrbündelreaktor, in dem eine langsame Strömung mittlere Verweilzeiten von ca. 1 Stunde erzeugt.

Aus der mit dem Dekanter abgetrennten Globinlösung wird das Protein durch Ultrafiltration entfernt und das Permeat erneut zur Häminfällung genutzt.

Als kritische Denaturierungstemperatur wurde für die relevanten Verfahrensbedingungen 75 °C über etwa 10 min ermittelt. Die Säurespaltung eines thermisch denaturierten Hämoglobins ist nicht möglich.

## Patentansprüche

1. Verfahren zur Gewinnung von Hämin
aus Schlachtblut,
**dadurch gekennzeichnet, daß**
das Schlachtblut zur Trennung des Hämin vom Globinanteil einer Säurebehandlung bei pH 1,2 - 3 bei 45 - 80 °C unterzogen und anschließend das Hämin vom Globin getrennt wird.

2. Verfahren nach Anspruch 1,
**dadurch gekennzeichnet, daß** vor der Säurebehandlung das Schlachtblut in Dickblut und eine Plasmafraktion getrennt, und das Dickblut der Säurebehandlung zugeführt wird.

3. Verfahren nach Anspruch 1 oder 2,
**dadurch gekennzeichnet, daß** die Säurebehandlung bei pH 1,3 - 2,0 bei einer Temperatur von 60 - 75 °C durchgeführt wird.

4. Verfahren nach mindestens einem der Ansprüche 1 bis 3,
**dadurch gekennzeichnet, daß** das Hämin vom Globin nach einer Agglomerationszeit von 15 Minuten bis 2 Stunden abgetrennt wird.

5. Verfahren nach Anspruch 4,
**dadurch gekennzeichnet, daß** eine Agglomerationszeit von 30 Minuten bis 1,5 Stunden eingehalten wird.

6. Verfahren nach mindestens einem der Ansprüche 1 bis 5,
**dadurch gekennzeichnet, daß** bei der Säurebehandlung eine Hämoglobinkonzentration von 2 bis 10 % eingestellt wird.

7. Verfahren nach mindestens einem der Ansprüche 1 bis 6,
**dadurch gekennzeichnet, daß** das Hämin vor der Trocknung einer Ultraschall-Behandlung unterzogen wird.

8. Verfahren nach mindestens einem der Ansprüche 1 bis 7,
**dadurch gekennzeichnet, daß** es kontinuierlich betrieben wird.

9. Verfahren nach mindestens einem der Ansprüche 1 bis 8,
**dadurch gekennzeichnet, daß** die heiße, saure Globinlösung, von Globin gereinigt, einem Kreislaufbetrieb zugeführt wird.

10. Hämin, erhältlich durch ein Verfahren nach mindestens einem der Ansprüche 1 bis 9,
**dadurch gekennzeichnet, daß** es 20 bis 80 % Globin enthält.

11. Verwendung von Hämin, erhätlich durch ein Verfahren nach mindestens einem der Ansprüche 1 bis 9,
für die Eisenfortifikation von Lebensmitteln.

## Claims

1. Method of obtaining haemin from slaughter blood,
***characterised in that***
the slaughter blood is subjected to an acid treatment at pH 1.2 - 3 at 45 - 80 °C in order to separate the haemin from the globin portion and subsequently the haemin is separated from the globin.

2. Method according to claim 1,
**characterised in that** the slaughter blood is separated into thick blood and a plasma fraction before the acid treatment and the thick blood is supplied to the acid treatment.

3. Method according to claim 1 or 2,
**characterised in that** the acid treatment is implemented at pH 1.3 - 2.0 at a temperature of 60 - 75 °C.

4. Method according to at least one of the claims 1 to 3,
**characterised in that** the haemin is separated from the globin after an agglomeration time of 15 minutes to two hours.

5. Method according to claim 4,
**characterised in that** an agglomeration time of 30 minutes to 1.5 hours is maintained.

6. Method according to at least one of the claims 1 to 5,
**characterised in that**, during the acid treatment, a haemoglobin concentration of 2 to 10% is set.

7. Method according to at least one of the claims 1 to 6,
**characterised in that** the haemin is subjected to an ultrasound treatment before drying.

8. Method according to at least one of the claims 1 to 7,
**characterised in that** it is operated continuously.

9. Method according to at least one of the claims 1 to 8,
**characterised in that** the hot, acid globin solution, cleaned of globin, is supplied to a circulation operation.

10. Haemin obtainable by means of a method according to at least one of the claims 1 to 9,
**characterised in that** it contains 20 to 80% globin.

11. Use of haemin, obtainable by means of a method according to at least one of the claims 1 to 9,
for the iron fortification of foodstuffs.

## Revendications

1. Procédé de production d'hémine à partir de sang d'abattoir,
**caractérisé en ce que**
le sang d'abattoir est soumis en vue de la séparation de l'hémine de la fraction globine, à un traitement acide à pH 1,2-3 à 45-80°C et ensuite l'hémine est séparée de la globine.

2. Procédé selon la revendication 1,
**caractérisé en ce qu'**
avant le traitement acide, le sang d'abattoir est séparé en sang coagulé et ou une fraction de plasma, et le sang coagulé est amené au traitement acide.

3. Procédé selon la revendication 1 ou la revendication 2,
**caractérisé en ce que**
le traitement acide est effectué à pH 1,3-2,0 à une température de 60 à 75°C.

4. Procédé selon au moins l'une quelconque des revendications 1 à 3,
**caractérisé en ce que**
l'hémine est séparée de la globine après un temps d'agglomération allant de 15 minutes à 2 heures.

5. Procédé selon la revendication 4,
**caractérisé en ce qu'**
on maintient un temps d'agglomération allant de 30 minutes à 1,5 heures.

6. Procédé selon au moins l'une quelconque des revendications 1 à 5,
**caractérisé en ce qu'**
au cours du traitement acide, on ajuste une concentration en hémoglobine de 2 à 10 %.

7. Procédé selon au moins l'une quelconque des revendications 1 à 6,
**caractérisé en ce qu'**
on soumet l'hémine avant le séchage à un traitement par ultrasons.

8. Procédé selon au moins l'une quelconque des revendications 1 à 7,
**caractérisé en ce qu'**
il est effectué en continu.

9. Procédé selon au moins l'une quelconque des revendications 1 à 8,
**caractérisé en ce que**
la solution de globine chaude, acide, purifiée par la globine, est amenée au fonctionnement en circuit.

10. Hémine que l'on peut produire selon un procédé selon au moins l'une quelconque des revendications 1 à 9,
**caractérisée en ce qu'**
elle contient de 20 à 80 % de globine.

11. Utilisation de l'hémine, que l'on peut produire selon un procédé selon au moins l'une quelconque des revendications 1 à 9,
pour fortifier en fer les aliments.
